# EUROPÄISCHE PATENTSCHRIFT

(11) **EP 1 876 168 B1**
(45) Veröffentlichungstag und Bekanntmachung des Hinweises auf die Patenterteilung: **04.03.2015**
(21) Anmeldenummer: 07110343.6
(22) Anmeldetag: 15.06.2007
(51) Int. Cl.: C07C 231/12, C07C 231/20, C07C 237/04, C07C 271/22, C07D 303/48

(54) **Verfahren zur Herstellung von beta-Amino-alpha-hydroxy-carbonsäureamiden**
Method for manufacturing beta amino alpha hydroxy carboxylic acid amides
Procédé destiné à la fabrication d'amides d'acides béta-amino-alpha-hydroxy-carbone

(30) Priorität: 04.07.2006 DE 102006031202
(43) Veröffentlichungstag der Anmeldung: 09.01.2008
(73) Patentinhaber: Evonik Degussa GmbH, 45128 Essen (DE)
(72) Erfinder: Knaup, Günter, 63486 Bruchköbel (DE)

(56) Entgegenhaltungen:
- WO-A-2005/082892
- WO-A1-2007/109023
- WO-A2-02/18369
- DE-C- 583 243
- AVAKYAN, A.S. ET AL: PHARMACEUTICAL CHEMISTRY JOURNAL, Bd. 31, Nr. 4, 1997, Seiten 178-181, XP002451480
- PUNNIYAMURTHY, T. ET AL: TETRAHEDRON LETTERS, Bd. 38, Nr. 25, 1997, Seiten 4463-4466, XP002451481

## Beschreibung

Die vorliegende Erfindung ist auf ein Verfahren zur Herstellung von β -Amino-α-hydroxycarbonsäureamiden gerichtet. Das Verfahren arbeitet mit Epoxy-carbonsäureamiden der Formel 2, 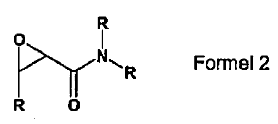 welche mit Ammoniak umgesetzt werden.

β-Amino-α-hydroxy-carbonsäureamide sind wichtige Intermediate für die chemische Synthese. Sie können beispielsweise weiter umgesetzt werden zu 3-Amino-2-ketamiden. Eine Vielzahl neuerer Proteasehemmer enthält als C-terminale Bausteine 3-Amino-2-ketoamide. Beispiele dafür sind Calpin-Inhibitoren (WO 95/00535), Thrombin Inhibitoren (J. Cacciola et al, Tetrahedron Lett. 38, 5741 (1997)) und ganz besonders eine Vielzahl von Hepatitis C Virus (HCV) Protease Inhibitoren wie z.B. in WO 05/087731, WO 05/058821 aufgeführt. Von letzteren sei beispielhaft VX-950 genannt (WO 01/74768, WO 02/18369), das sich bereits in der fortgeschrittenen klinischen Entwicklung befindet.

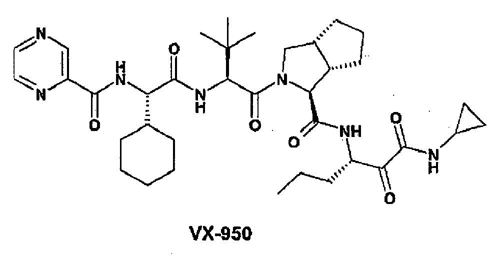

Das gebräuchlichste Verfahren zur Herstellung von 3-Amino-2-ketamiden besteht in der Oxidation der entsprechenden β -Amino-α-hydroxy-carbonsäureamide:

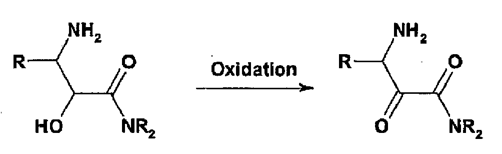

Für die Herstellung dieser β-Amino-α-hydroxy-carbonsäureamide werden verschiedene Verfahren angewandt, die im Folgenden beispielhaft für 3-Amino-2-hydroxy-hexansäure aufgeführt sind:
1. Kondensation von Nitroalkanen mit Glyoxalsäure, Reduktion der Nitrogruppe und nachfolgende Überführung der Säure in das Amid (K.X. Chen , J.Med. Chem. 49, 567, (2006); ibid. 995): 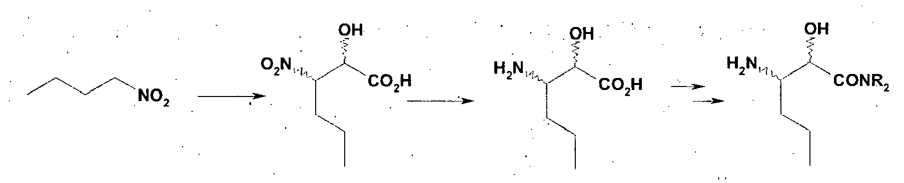 Nachteilig an diesem Verfahren ist, dass die komplette Palette der möglichen stereoissomeren Verbindungen erhalten werden, die nach Oxidation der Hydroxygruppe zu Mischungen von diastereomeren Verbindungen führen, die in den meisten Fällen nicht getrennt werden können. Falls doch, ist dies nur mit chromatographischen Methoden möglich (K.X. Chen , J.Med. Chem. 49, 995 (2006)).
2. Überführung von geschützten Aminosäuren in die Aldehyde, Umsetzung zu den Cyanhydrinen, deren Verseifung zur Säure und nachfolgende Überführung in die Amide (z.B.: WO 01/74768, WO02/18369, WO 05/087731, S.-H. Chen, Lett. Drug Design & Discovery, 2005, 118). 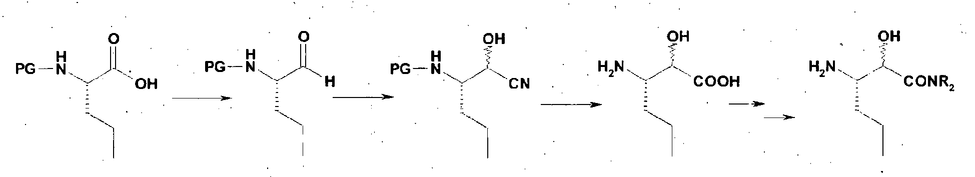 Nachteilig an diesem Verfahren ist dass man von hochpreisigen Ausgangsmaterialen ausgeht und Mischungen der diastereomeren Aminoalkohole erhält, die bei den folgenden Syntheseschritten eine Isolierung von Zwischenprodukten erschweren können. Weiterhin nachteilig ist, dass zwangsläufig die freien Aminohydroxysäuren erhalten werden, zu deren Überführung in die Amide eine erneute Einführung einer N-Schutzgruppe und in der Regel eine Aktivierung der Säurefunktion erforderlich ist.
3. Durch enantioselektive Aminohydroxylierung (Sharpless, Angew. Chem. 109, 2752 (1997); WO 97/46516, WO 98/42657) von Acrysäureestern (WO 05/087731, US 2005/0197301): 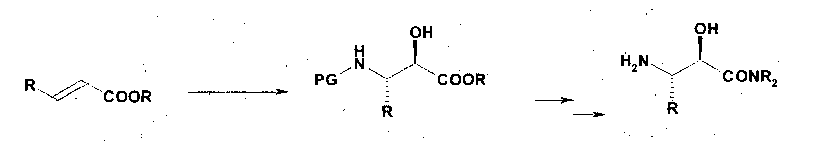 Nachteilig an diesem Verfahren ist, dass große Mengen chiraler Katalysatoren und Osmiumoxid (toxisch!) als Oxidationsmittel benötigt werden.
4. Primäre Amide können in Analogie zu Punkt 2 durch Umsetzung der Aldehyde mit Isonitrilen und Essigsäure hergestellt werden (J.G. Catalano , Biorg. Med. Chem Lett., 14, 719 (2004); D.G. Barrett, ibid. 2543; L. Banfi, Molecular Diversity, 6, 227 (2003), WO 05/087731, US 2005/0197301): 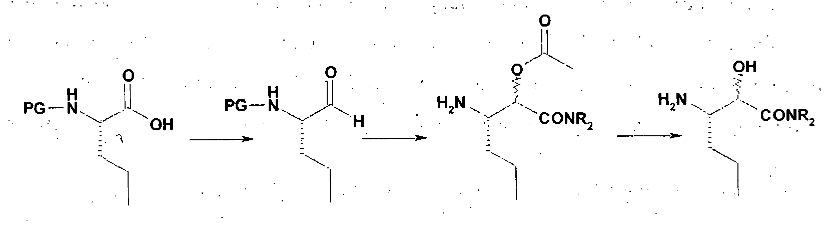 Neben den unter 2. genannten Nachteilen kommt bei dieser Variante noch hinzu, dass die benötigten Isonitrile oft nicht kommerziell erhältlich sind, sondern aufwendig hergestellt werden müssen.
5. Eine weitere Möglichkeit besteht in der regioselektiven Ringöffnung von Epoxyamiden mittels Azid und nachfolgende Reduktion zu Aminoverbindungen (J. Cacciola, Tetrahedron Lett. 38, 5741 (1997); US 2003/0153788, K.B. Sharpless, J.Org. Chem. 50, 5696 (1985)).

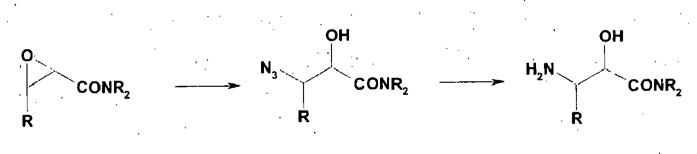

Nachteilig an dieser Variante ist, dass zur Ringöffnung Azide eingesetzt werden müssen, die sicherheitstechnisch problematisch sind und zur Überführung in die Amine ein zusätzlicher Reduktionsschritt erforderlich ist..

Für dieses Verfahren können auch enantiomerenreine Epoxide eingesetzt werden, die dann direkt einen diastereomerenreinen Aminoalkohol liefern. Für die Herstellung enantiomerenreiner Epoxide muss aber entweder der entsprechenden Allylalkohol nach Sharpless epoxidiert werden (J. Cacciola, Tetrahedron Lett. 38, 5741 (1997), US 2003/0153788) oder für die Epoxidierung der entsprechenden Acrylsäuren müssen Lanthanidkomplexe (T. Ohshima, Tetrahedron 59, 10485 (2003)) verwendet werden. Eine direkte asymmetrische Synthese von Epoxidamiden ist durch Umsetzung von chiralen S-Alkyl-thioglycolsäureamiden mit Aldehyden möglich (WO 03/08707).

Es ist darüber hinaus auch bekannt, dass Epoxy-carbonsäuren und Ester direkt mit Ammoniak oder Aminen, bevorzugt Benzylamin, geöffnet werden können. Die freien Säuren werden dabei ausschließlich durch Angriff am C-2-Atom geöffnet (z.B.: Y. Liwschitz, J. Chem. Soc., 1961, 1116). Eine Ausnahme bildet die 3-Phenylglycidsäure, die mit Ammoniak überwiegend zum Phenylisoserin reagiert (WO 03/003804). Durch Zusatz von mindestens stöchiometrischen Mengen Ti(O-iPr)₄ kann bei der Reaktion aliphatischer Säuren ebenfalls eine Umkehr der Selektivität erreicht werden (K.B. Sharpless, J.Org.Chem. 50, 1560 (1985)). Ester werden dagegen bevorzugt durch Angriff an das C-3-Atom geöffnet (J. Chem. Soc. Perkin I, 1980, 1618). Die Ester werden dabei zumindest teilweise in die entsprechenden Amide überführt. Eine direkte Umsetzung von N-substituierten Epoxy-amiden mit Ammoniak hat bisher noch keine Anwendung gefunden, offensichtlich weil die Amide in der Reaktivitätsskala zwischen den Estern und den Säuren rangieren und man erwarten konnte hier Gemische aus 2- und 3-substituierten Aminohydroxyamiden zu erhalten. Bei Umsetzungen mit Ammoniak ist unter den erforderlichen Reaktionsbedingungen darüber hinaus mit Umamidierungen zu rechnen. Daher wurde bevorzugt die bereits oben beschriebene Umsetzung mit Aziden angewandt.

Während die Umsetzungen von beta-Arylglycidestern mit Ammoniak und Amine gut untersucht wurde (W. Tack, Arch. Pharm. 312, 138 (1979) und cit. lit; E. Kamandi, Arch. Pharm. 308, 135 (1975) und cit. lit., US 6,025,516, WO 06/008170, ist über die Umsetzung von aliphatischen Glycidestern nur wenig bekannt.

Angesichts dieses Standes der Technik bestand die Aufgabe, einen einfachen Zugang zu enantiomerenreinen 3-Amino-2-hydroxy-amiden der Formel (I) zu finden, der von einfach verfügbaren Ausgangsmaterialen ausgeht, einfach im technischen Maßstab durchführbar ist und keine besondere Anforderungen an die technischen Einrichtungen stellt. Insbesondere sollte das anvisierte Verfahren vom ökonomischen wie ökologischen Standpunkt aus betrachtet den Verfahren des Standes der Technik überlegen sein.

Dadurch, dass man in einem Verfahren zur Herstellung von Verbindungen der allgemeinen Formel (I) 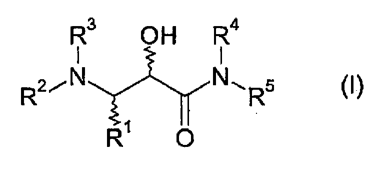 worin
R¹
(C₁-C₈)-Alkyl ist, R², R³, R⁴ sind, und R⁵ cyclopropyl ist
Epoxy-carbonsäureamide der allgemeinen Formel (II) 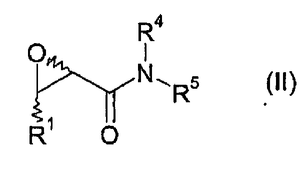 worin
R¹, R⁴, R⁵ die oben angegebene Bedeutung annehmen, mit Ammoniak umsetzt, gelang man völlig überraschend und dafür nicht minder vorteilhaft zur Lösung der gestellten Aufgabe. Die so erhaltenen Verbindungen der allgemeinen Formel (I) werden in hohen Ausbeuten, wider Erwarten hoher Regioisomerenreinheit und unter nahezu vollständigem Erhalt der Diastereomerenreinheiten erhalten.

Verbindungen, in denen R' einen linearen C₃-Alkyl-Rest darstellt, sind zu bevorzugen.
Besonders bevorzugt ist das Verfahren auf die 3-Propyl-oxirancarbonsäure-N-cyclopropylamid gemäß Formel 3 anwendbar.

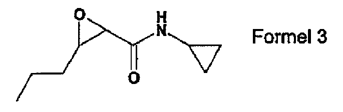

In einer besonderen Ausgestaltung bezieht sich die vorliegende Erfindung auf ein Verfahren zur Herstellung von enantiomerenangereicherten Verbindungen der allgemeinen Formel (I), in dem man enantiomerenangereicherte Verbindungen der Formel (II) einsetzt oder die ansonsten entstehenden racemischen diastereomerenangereicherten Verbindungen der Formel (I) als Salz mit chiralen organischen Säuren enantioselektiv kristallisiert.

Die erfindungsgemäße Reaktion kann in einem dem Fachmann für diesen Zweck in Frage kommendem Lösungsmittel durchgeführt werden. Die Umsetzung wird vorzugsweise in einem Lösungsmittel ausgewählt aus der Gruppe bestehend aus Wasser, wasserhaltigen und nicht wasserhaltigen organischen Lösungsmitteln durchgeführt. Bevorzugt wird die Umsetzung in protischen Lösungsmitteln wie z.B. Alkoholen oder Wasser und ganz besonders in Mischungen daraus (wie z.B. Ethanol und konz. wässriger Ammoniak) durchgeführt.

Die Umsetzung wird vorzugsweise bei einer Temperatur von 0 - 200 °C, vorzugsweise 20 - 150°C und ganz besonders von 50 - 100 °C in einem Druckgefäß beim sich einstellendem Eigendruck durchführt.

Die Epoxy-carbonsäureamide der Formel (II) können nach dem Fachmann bekannten Verfahren hergestellt werden, z.B. einfach aus den entsprechenden Säuren und den Aminen der Formel HNR⁴R⁵. Zur Aktivierung der Epoxycarbonsäuren können die aus der Peptidchemie bekannten Methoden (z.B. Houben-Weyl Bd15, Synthese von Peptiden, Thieme Verlag Stuttgart 1974) herangezogen werden. Bevorzugt werden diese Verbindungen allerdings wie in der US 2003/0153788 beschrieben mittels gemischter Anhydride und hier ganz besonders mittels Pivaloylchlorid hergestellt. Besonders bevorzugt werden Alkalimetallsalze der Epoxycarbonsäuren, ganz besonders Kaliumsalze, für die Umsetzung mit den Säurechloriden verwendet.

Die Epoxycarbonsäuren können als 4 verschiedene Stereoisomern vorkommen. Das erfindungsgemäße Verfahren verläuft vorzugsweise so, dass aus den trans-Epoxidamiden der Formel (II) stereoselektiv die erythro-Amino-hydroxyverbindungen der Formel (I) entstehen, während aus den cis-Verbindungen selektiv die entsprechenden threo-Verbindungen resultieren. Bei Einsatz der enantiomerenreinen Epoxy-carbonsäureamide der Formel (II) resultieren die entsprechenden Amino-hydroxyverbindungen der Formel (I), in denen die Konfiguration am C-Atom 3 gegenüber der der Epoxyverbindung vertauscht ist.

Hoch enantiomerenangereicherte nahezu reine trans- und cis-Epoxy-carbonsäureamide (II) können unter Erhalt der Stereochemie aus den entsprechenden Epoxycarbonsäuren nach den oben angesprochenen Methoden hergestellt werde. Die Ausgangsstoffe wiederum sind stereoselektiv durch achirale Epoxidierung (EP 618202), K.B. Sharpless, J.Org.Chem. 50, 1979 (1985)) der entsprechenden E- oder Z Acrylsäuren bzw. deren Ester zugänglich. Darüberhinaus kann, wie z.B. in der US 2003/0153788 beschrieben, eine enantioselektive Epoxidierung zu den enantiomerenangereicherten Epoxy-alkoholen und nachfolgende Oxidation zu den Epoxycarbonsäuren durchgeführt werden.

Eine weitere gebräuchliche Methode zur Herstellung von Epoxycarbonsäure-derivaten ist die Darzen-Glycidestersynthese. Ausgehend von Aldehyden und Halogenessigsäureestern werden überwiegend die trans-Verbindungen gebildet. Ähnliche Ergebnisse werden bei der Umsetzung von Carbonylverbindungen mit Schwefel-yliden erhalten (WO 03/087075) erhalten. In vielen Fällen kann dann durch Kristallisation der entsprechenden Epoxycarbonsäuresalze die reinen trans-Verbindungen erhalten werden. Dies hat den weiteren Vorteil, dass diese Salze direkt mit Säurechloriden zu den gemischten Anhydriden und diese wiederum ggf. in einem Topf in die Amide umgesetzt werden können.

Wie schon angedeutet können enantiomerenreinen und diastereomerenreinen Derivate der Verbindungen der allgemeinen Formel (I), besonders bevorzugt der erythro-Verbindungen davon, durch Salzpaarbildung mit chiralen Säuren, wie z.B. Hydroxysäuren (z.B.Mandelsäure, Weinsäure, Dibenzoyl-weinsäure, Ditoluolyl-weinsäure, Milchsäure), N-Acyl-aminosäuren (z.B. Benzyloxycarbonyl-L-phenylalanin, Acetyl-L-phenylalanin) oder Sulfonsäuren (z.B. Camphersulfonsäure) hergestellt werden. Bevorzugt werden Salze der Verbindungen der Formel (I) mit Mandelsäure und Benzyloxycarbonyl-phenylalanin gebildet. Bevorzugt ist demnach ebenfalls ein erfindungsgemäßes Verfahren bei dem man die entstehenden racemischen diastereomerenangereicherten Verbindungen der Formel (I) als Salz mit chiralen organischen Säuren enantioselektiv kristallisiert.

Die Salzbildung wird bevorzugt mit 0,2 - 2,0 besser 0,3 - 1,0, ganz bevorzugt 0,5 - 0,6 Äquivalenten der chiralen Säure durchgeführt. Als Lösungsmittel werden, je nach Löslichkeit der Salze, organische Lösungsmittel wie Alkohole, Ester, Ether, Kohlenwasserstoffe eingesetzt. Besonders bevorzugt werden Alkohole wie Ethanol und Isopropanol und Essigsäureester wie Ethylacetat, n-Butylacetat und Isopropylacetat eingesetzt. Zur Erhöhung der Löslichkeit kann den Lösungsmitteln noch etwas Wasser zugesetzt werden. Bevorzugt werden die Verbindungen der Formel (I) und die chiral Säure in der Hitze gelöst und die Salze durch Abkühlen kristallisiert.
Die freien Basen der Verbindungen der Formel (I) können aus den Salzen durch bekannte Verfahren wie z.B. Säure-Base-Extraktion gewonnen werden. Auch eine Umsalzung mit stärkeren Säuren, wie z.B. Salzsäure ist möglich.

Als (C₁-C₈)-Alkylreste sind anzusehen Methyl, Ethyl, *n*-Propyl, Isopropyl, *n*-Butyl, Isobutyl, *sec-*Butyl, *tert*-Butyl, Pentyl, Hexyl, Heptyl oder Octyl samt aller ihrer Bindungsisomeren.
Der Rest (C₁-C₈)-Alkoxy entspricht dem Rest (C₁-C₈)-Alkyl mit der Maßgabe, dass dieser über ein Sauerstoffatom an das Molekül gebunden ist.
Als (C₂-C₈)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an.
Eine (C₂-C₈)-Alkylenbrücke ist eine Kohlenstoffkette mit drei bis acht C-Atomen, wobei diese Kette über zwei verschiedene C-Atome an das betrachtete Molekül gebunden ist. Diese kann einen gesättigten oder ungesättigten carbocyclischen oder heterocyclischen Ring bedeuten, mit 1-4 Stickstoff-, Silicium-, Schwefel-, Phosphor- oder Sauerstoffatomen im Ringsystem.
Die in den vorangehenden Absätzen beschriebenen Reste können einfach oder mehrfach mit Halogenen und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an den Rest gebunden sind.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Siatomhaltige Reste substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein (C₃-C₈)-Cycloalkyl-(C₁-C₈)-Alkylrest bezeichnet einen wie oben dargestellten Cycloalkylrest, welcher über einen wie oben angegebenen Alkylrest an das Molekül gebunden ist.

(C₁-C₈)-Acyloxy bedeutet im Rahmen der Erfindung einen wie oben definierten Alkylrest mit max. 8 C-Atomen, welcher über eine COO-Funktion an das Molekül gebunden ist.

(C₁-C₈)-Acyl bedeutet im Rahmen der Erfindung einen wie oben definierten Alkylrest mit max. 8 C-Atomen, welcher über eine CO-Funktion an das Molekül gebunden ist.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, wie z.B. Indenylsysteme, welche ggf. mit Halogen, (C₁-C₈)-Alkyl, (C₁-C₈)-Alkoxy, NH₂, NH(C₁-C₈)-Alkyl, N((C₁-C₈)-Alkyl)₂, OH, CF₃, NH(C₁-C₈)-Acyl, N((C₁-C₈)-Acyl)₂, (C₁-C₈)-Acyl, (C₁-C₈)-Acyloxy, substituiert sein können.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl. Dieser Rest kann mit den gleichen Resten substituiert sein wie der oben genannte Arylrest.

Unter einem (C₄-C₁₉)-Heteroaralkyl wird ein dem (C₇-C₁₉)-Aralkylrest entsprechendes heteroaromatisches System verstanden.

Als Halogene (Hal) kommen Fluor, Chlor, Brom und Iod in Frage.

Unter dem Begriff enantiomerenangereichert, enantiomer angeriechert oder Enantiomerenüberschuss wird im Rahmen der Erfindung der Anteil eines Enantiomers im Gemisch mit seiner optischen Antipode in einem Bereich von >50 % und <100 % verstanden. Der ee-Wert berechnet sich wie folgt: ([Enantiomer1]-[Enantiomer2])/([Enantiomer1]+[Enantiomer2])=ee-Wert

Der Begriff diastereomerenangereichert bezeichnet den Anteil eines Diastereomers im Gemisch mit den anderen möglichen Diastereomeren der betrachteten Verbindung.

Die hier genannten Strukturen von Verbindungen umfassen und offenbaren alle theoretisch möglichen Diastereomere bzw. Enantiomere, die durch Variation der Konfigurationen an den entsprechenden C-Atomen auftreten können.

### Beispiele:

### Beispiel 1: trans-3-n-Propyl-oxirancarbonsäure-N-cyclopropylamid

200 g trans-3-n-Propyl-oxirancarbonsäure Kaliumsalz werden in 2 l Aceton suspendiert, mit 120 g Triethylamin versetzt und auf 3°C abgekühlt. Dann werden 146 g Pivaloylchlorid zugegeben, 20 min bei 20°C nachgerührt und dann auf 3°C abgekühlt. Hierzu werden 82 g Cyclopropylamin in 175 ml Aceton gegeben. Nach 20 min Nachrühren wird das Lösungsmittel i.Vak. abgezogen und der Rückstand in 2 l Toluol und 400 ml Wasser gelöst und der pH Wert auf 10 gestellt. Die wässrige Phase wird abgetrennt und die organische Phase mit 300 ml Wasser versetzt und der pH-Wert mit Salzsäure auf 2 gestellt. Die organische Phase wird abgetrennt und i.Vak. eingeengt. Man erhält 175 g trans-3-n-Propyl-oxirancarbonsäure-N-cyclopropylamid als hellbraunes Öl. 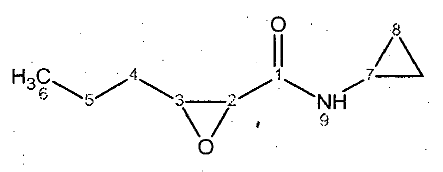 ¹H-NMR: von N-Cyclopropyl-*trans*-propyloxirancarboxamid (500 MHz, DMSO, 303 K):

| **δ / ppm** | **Multiplizität** | **Intensität** | **Zuordnung** |
|---|---|---|---|
| 0.40 - 0.65 | m | 4 | 8 |
| 0.91 | t | 3 | 6 |
| 1.42 / 1.54 | m / m | 1 / 1 | 4 |
| 1.24 | m | 2 | 5 |
| 1.60 | s (broad) | 2 | NH₂ |
| 2.64 | m | 1 | 7 |
| 2.97 | m | 1 | 3 |
| 3.10 | d | 1 | 2 |
| 7.98 | d | 1 | NH 9 |

### Beispiel 2: erythro-3-Amino-2-hydroxy-hexansäure-N-cyclopropylamid

### a) mit wasserfreiem Ammoniak

183 g trans-3-n-Propyl-oxirancarbonsäure-N-cyclopropylamid werden in 1683 g 10,5 Gew.-%igen ethanolischen Ammoniak gelöst und im verschlossenen Autoklav 6 Std. auf 100°C erhitzt. Danach wird auf Raumtemperatur abgekühlt, das Lösungsmittel i.Vak. entfernt. Der Rückstand wird in 700 ml Toluol suspendiert, wieder weitestgehend eingeengt und mit frischem Toluol aufgefüllt. Durch Erhitzen zum Rückfluss wird der Rückstand gelöst. Nach Abkühlen auf 0°C und abfiltrieren erhält man 136 g erythro-3-Amino-2-hydroxy-hexansäure-N-cyclopropylamid.
Schmp.:101 - 104°C 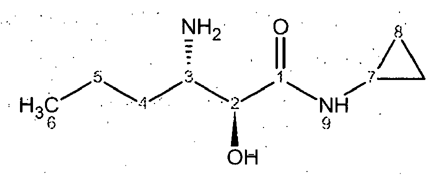 ¹H-NMR von S,S/R,R-3-Amino-2-hydroxy-hexansäure-N-cyclopropylamid (500 MHz, DMSO, 303 K)

| **δ / ppm** | **Multiplitität** | **Intensität** | **Zuordnung** |
|---|---|---|---|
| 0.40 - 0.65 | m | 4 | 8 |
| 0.83 | t | 3 | 6 |
| 1.13 / 1.43 | m / m | 1 / 1 | 4 |
| 1.24 | m | 2 | 5 |
| 1.60 | s (broad) | 2 | NH₂ |
| 2.65 | m | 1 | 7 |
| 2.79 | m | 1 | 3 |
| 3.69 | d | 1 | 2 |
| 5.31 | s (broad) | 1 | OH |
| 7.68 | d | 1 | NH 9 |

### b) mit wässrigem Ammoniak

161 g trans-3-n-Propyl-oxirancarbonsäure-N-cyclopropylamid werden in 405 g 25 Gew.-%igen Ammoniak gelöst und im verschlossenen Autoklav 3 Std. auf 90°C erhitzt. Abdestillieren des überschüssigen Ammoniaks i.Vak. liefert 576 g einer 44,3 gew.-%igen wässrigen Lösung, die direkt zur Racematspaltung eingesetzt werden kann.

### Beispiel 3: (2S,3S)-3-Amino-2-hydroxy-hexansäure-N-cyclopropylamid-(S)-Mandelsäuresalz

83,8 g erythro-3-Amino-2-hydroxy-hexansäure-N-cyclopropylamid und 34,4 g (S)-Mandelsäure werden bei 70°C in 1500 ml Isopropanol gelöst. Der nach Abkühlen auf 2°C sich bildende Niederschlag wird abfiltriert und 2 mal aus Isopropanol umkristallisiert. Man erhält 42 g (2S,3S)-3-Amino-2-hydroxy-hexansäure-N-cyclopropylamid-(S)-Mandelsäuresalz.
Schmp.:132 - 134°C
chirale HPLC: 0,3 % (R,R)-Isomeres

### Beispiel 4: (2S,3S)-3-Amino-2-hydroxy-hexansäure-N-cyclopropylamid-N-Benzyloxycarbonyl-L-phenylalanin Salz

18,6 g erythro-3-Amino-2-hydroxy-hexansäure-N-cyclopropylamid und 15,9 g N-Benzyloxycarbonyl-L-phenylalanin werden bei Rückflußtemperatur in 400 ml Ethylacetat und 33 ml Wasser gelöst.. Der nach Abkühlen auf 0°C sich bildende Niederschlag wird abfiltriert und aus Ethanol umkristallisiert. Man erhält 16,7 g (2S,3S)-3-Amino-2-hydroxy-hexansäure-N-cyclopropylamid-N-benzyloxycarbonyl-L-phenylalanin-Salz.
Schmp.:195 - 197°C
chirale HPLC: R,R-Diastereomeres: < 0,3 % 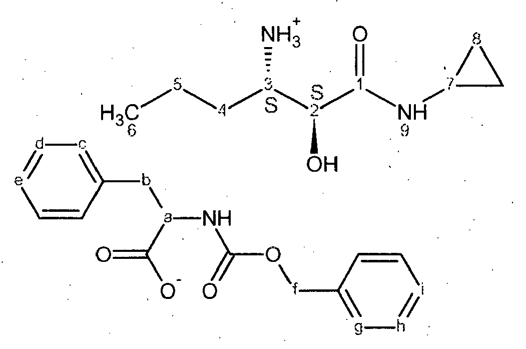 ¹H-NMR von (2S,3S)-3-Amino-2-hydroxy-hexansäure-N-cyclopropylamid * Z-Phenylalanine (500 MHz, DMSO, 303 K)

| **δ / ppm** | **Multiplizität** | **Intensität** | **Zuordnung** |
|---|---|---|---|
| 0.45 - 0.65 | m | 4 | 8 |
| 0.84 | t | 3 | 6 |
| 1.23 / 1.37 | m / m | 2 | 4 |
| 1.37 | m | 2 | 5 |
| 2.68 | m | 1 | 7 |
| 2.85 / 3.09 | dd / dd | 2 | b |
| 3.28 | m | 1 | 3 |
| 3.99 | m | 1 | a |
| 4.11 | d | 1 | 2 |
| 4.96 | dd | 2 | f |
| 6.78 | d | 1 | NH Z |
| 7.1 - 7.4 | m | 10 | 2 * Phe |
| 7.95 | d | 1 | NH 9 |

### Beispiel 5: (2S,3S)-3-Amino-2-hydroxy-hexansäure-N-cyclopropylamid Hydrochlorid

38,7 g (2S,3S)-3-Amino-2-hydroxy-hexansäure-N-cyclopropylamid-(S)-Mandelsäuresalz werden in 500 ml Isopropylacetat suspendiert und mit 11,8 g 37 5-iger Salzsäure versetzt. Nach 1 Std. Rühren bei Raumtemperatur wird die Hälfte des Lösungsmittels i.Vak. abdestilliert und durch frisches Isopropylacetat ersetzt. Nachdem dies nochmals wiederholt wurde, wird auf 60oC erhitzt, der Festsoff abfiltriert und mit warmen Isopropylacetat gewaschen. Man erhät 25,0 g (2S,3S)-3-Amino-2-hydroxy-hexansäure-N-cyclopropylamid Hydrochlorid.
Schmp.:212 - 215°C
HPLC: R,S / S,R-Diastereomere: < 0.1 %
chirale HPLC: R,R-Diastereomeres: < 0.3 %

## Patentansprüche

1. Verfahren zur Herstellung von Verbindungen der allgemeinen Formel (I) 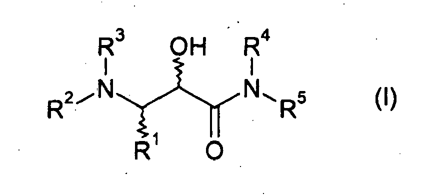 worin
R¹ = (C₁-C₈)-Alkyl
R², R³, R⁴ = H und
R⁵ = Cyclopropyl ist,
**dadurch gekennzeichnet, dass** man
Epoxy-carbonsäureamide der allgemeinen Formel (II) 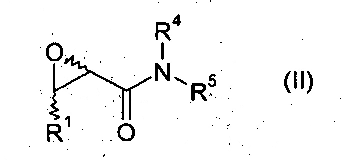 worin
R¹, R⁴, R⁵ die oben angegebene Bedeutung annehmen, mit Ammoniak umsetzt.

2. Verfahren nach Anspruch 1,
**dadurch gekennzeichnet, dass** man
enantiomerenangereicherte Verbindungen der allgemeinen Formel (I) herstellt, in dem man enantiomerenangereicherte Verbindungen der Formel (II) einsetzt.

3. Verfahren nach Anspruch 1, **dadurch gekennzeichnet, dass** man die entstehenden racemischen diastereomerenangereicherten Verbindungen der Formel (I) als Salz mit chiralen organischen Säuren enantioselektiv kristallisiert.

4. Verfahren gemäß Anspruch 3, **dadurch gekennzeichnet, dass** die chiralen, organischen Säuren, ausgewählt sind aus der Gruppe der Hydroxysäuren, N-Acylaminosäuren, Sulfonsäuren und Aminodicarbonsäuren.

5. Verfahren gemäß Anspruch 4, **dadurch gekennzeichnet, dass** als chirale organische Säure Mandelsäure, Weinsäure, Dibenzoylweinsäure, Ditoluolylweinsäure, Milchsäure, Benzyloxycarbonyl-L-phenylalanin, Acetyl-L-phenylalanin oder Camphersulphonsäure eingesetzt wird.

6. Verfahren nach Anspruch 1 und/oder 2,
**dadurch gekennzeichnet, dass** man
die Umsetzung in einem Lösungsmittel ausgewählt aus der Gruppe bestehend aus Wasser, wasserhaltigen und nicht wasserhaltigen organischen Lösungsmitteln durchführt.

7. Verfahren nach einem oder mehreren der Ansprüche 1 bis 5,
**dadurch gekennzeichnet, dass** man
die Umsetzung bei einer Temperatur von 0 - 200 °C, vorzugsweise 20 - 150°C und ganz besonders von 50 - 100 °C in einem Druckgefäß beim sich einstellendem Eigendruck durchführt.

## Claims

1. Process for preparing compounds of the general formula (I) 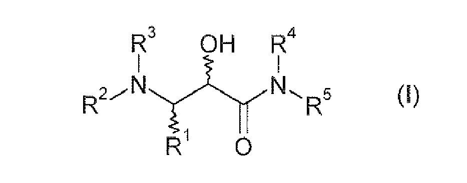 in which
R¹ = (C₁-C₈-alkyl),
R², R³, R⁴ = H and
R⁵ = cyclopropyl,
**characterized in that**
epoxycarboxamides of the general formula (II) 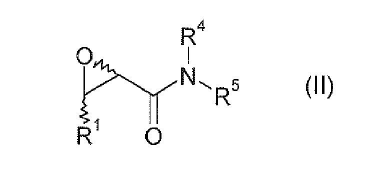 in which
R¹, R⁴, R⁵ are each as defined above are reacted with ammonia.

2. Process according to Claim 1,
**characterized in that**
enantiomerically enriched compounds of the general formula (I) are prepared by using enantiomerically enriched compounds of the formula (II).

3. Process according to Claim 1, **characterized in that** the racemic diastereomerically enriched compounds of the formula (I) formed are crystallized enantioselectively as the salt with chiral organic acids.

4. Process according to Claim 3, **characterized in that** the chiral organic acids are selected from the group of the hydroxy acids, N-acyl amino acids, sulphonic acids and aminodicarboxylic acids.

5. Process according to Claim 4, **characterized in that** the chiral organic acid used is mandelic acid, tartaric acid, dibenzoyltartaric acid, ditolyltartaric acid, lactic acid, benzyloxycarbonyl-L-phenylalanine, acetyl-L-phenylalanine or camphorsulphonic acid.

6. Process according to Claim 1 and/or 2,
**characterized in that**
the reaction is performed in a solvent selected from the group consisting of water, aqueous and nonaqueous organic solvents.

7. Process according to one or more of Claims 1 to 5,
**characterized in that**
the reaction is performed at a temperature of 0 - 200°C, preferably 20 - 150°C and very particularly of 50 - 100°C in a pressure vessel at the autogenous pressure which is established.

## Revendications

1. Procédé de fabrication de composés de formule générale (I) 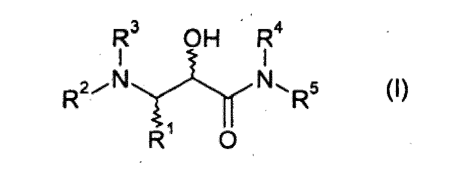 dans laquelle
R¹ = alkyle en (C₁-C₈),
R², R³, R⁴ = H et
R⁵ = cyclopropyle,
**caractérisé en ce que**
des amides d'acides époxy-carboxyliques de formule générale (II) 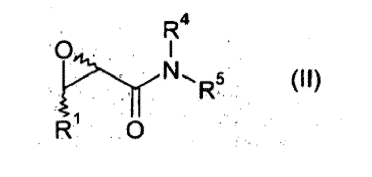 dans laquelle
R¹, ^{R4}, R⁵ ont la signification indiquée précédemment, sont mis en réaction avec de l'ammoniac.

2. Procédé selon la revendication 1, **caractérisé en ce que** des composés de formule générale (I) enrichis en énantiomères sont fabriqués en utilisant des composés de formule (II) enrichis en énantiomères.

3. Procédé selon la revendication 1, **caractérisé en ce que** les composés de formule (I) enrichis en diastéréomères racémiques formés sont cristallisés de manière énantiosélective sous la forme d'un sel avec des acides organiques chiraux.

4. Procédé selon la revendication 3, **caractérisé en ce que** les acides organiques chiraux sont choisis dans le groupe des hydroxyacides, des N-acylaminoacides, des acides sulfoniques et des acides aminodicarboxyliques.

5. Procédé selon la revendication 4, **caractérisé en ce que** l'acide mandélique, l'acide tartrique, l'acide dibenzoyltartrique, l'acide ditoluolyltartrique, l'acide lactique, la benzyloxycarbonyl-L-phénylalanine, l'acétyl-L-phénylalanine ou l'acide camphre-sulfonique est utilisé en tant qu'acide organique chiral.

6. Procédé selon la revendication 1 et/ou 2, **caractérisé en ce que** la réaction est réalisée dans un solvant choisi dans le groupe constitué par l'eau, les solvants organiques contenant de l'eau et ne contenant pas d'eau.

7. Procédé selon une ou plusieurs des revendications 1 à 5, **caractérisé en ce que** la réaction est réalisée à une température de 0 à 200 °C, de préférence de 20 à 150 °C et tout particulièrement de 50 à 100 °C, dans un récipient sous pression à la pression propre qui s'ajuste.
